# EUROPEAN PATENT APPLICATION

(11) **EP 4 840 852 A1**
(43) Date of publication of application: **18.11.2026**
(21) Application number: 24923441.0
(22) Date of filing: 08.11.2024
(51) Int. Cl.: C07D 253/07, A61K 31/53, A61P 13/12, A61P 3/10, A61P 17/02, A61P 37/06, A61P 9/12

(54) **DIPHENYLTRIAZINE COMPOUND, PREPARATION METHOD THEREFOR AND USE THEREOF**

(30) Priority: 06.02.2024 CN 202410169645; 31.05.2024 CN 202410701022
(71) Applicant: SHIJIAZHUANG NO. 4 PHARMACEUTICAL CO., LTD, Shijiazhuang, Hebei 050035 (CN); HEBEI GUOLONG PHARMACEUTICAL CO., LTD, Shijiazhuang, Hebei 052160 (CN)
(72) Inventor: SUN, Lijie, Shijiazhuang, Hebei 050035 (CN); SUN, Xiaofei, Shijiazhuang, Hebei 050035 (CN); YANG, Xian, Shijiazhuang, Hebei 050035 (CN); CAO, Liu, Shijiazhuang, Hebei 050035 (CN); LI, Lei, Shijiazhuang, Hebei 050035 (CN); ZHU, Shujie, Shijiazhuang, Hebei 050035 (CN); GUO, Zhiqiang, Shijiazhuang, Hebei 050035 (CN); ZHAO, Fanghong, Shijiazhuang, Hebei 050035 (CN); LU, Xiaobing, Shijiazhuang, Hebei 050035 (CN); HOU, Haiting, Shijiazhuang, Hebei 050035 (CN)
(74) Representative: Isarpatent
(86) International application number: PCT/CN2024/130940
(87) International publication number: WO 2025/167237

(57) **Abstract**

The present application relates to the field of chemical medicine, and particularly to a diphenyltriazine compound, a preparation method therefor and a use thereof. The diphenyltriazine compound does not have a PGI2 backbone, has strong selectivity and affinity for a PGI2 receptor, and has good target selectivity as compared with a PGI2 analogue. Experiments have proven that the in-vivo efficacy of the compound is superior to that of Selexipag, and the toxicity of the compound is significantly lower than that of Selexipag. Therefore, the compound can be used as a pulmonary arterial hypertension therapeutic drug having a higher clinical value and development prospect.

## Description

### CROSS-REFERENCE TO RELATED APPLICATIONS

This application claims priority to Chinese Patent Application No. 202410169645.1, filed on February 6, 2024 and Chinese Patent Application No. 202410701022.4, filed on May 31, 2024, the entire contents of all of which are hereby incorporated by reference.

### FIELD OF THE INVENTION

The application relates to the field of chemical medicine, and particularly to a diphenyltriazine compound, a preparation method therefor and a use thereof.

### BACKGROUND OF THE INVENTION

Pulmonary arterial hypertension (PAH) is a disease primarily characterized by vasospasm, intimal hyperplasia, and remodeling of the small pulmonary arteries. The vascular proliferation and remodeling of the small pulmonary arteries lead to a progressive increase in pulmonary vascular resistance, ultimately resulting in right heart failure and death. PAH has been ranked as the third most common cardiovascular disease, with a prevalence second only to hypertension and coronary heart disease. It has become a serious public health issue threatening human physical and mental health, and has been included by the World Health Organization (WHO) in the scope of major chronic diseases worldwide surveillance.

Prostaglandin I2 (PGI2) is a substance produced in the body from arachidonic acid via prostaglandin H2 (PGH2). PGI2 deficiency can lead to PAH. Currently, marketed PGI2 receptor agonists include epoprostenol, beraprost, iloprost, and others, all of which are PGI2 analogue. However, due to the very short biological half-life of PGI2 and its poor target selectivity, it is difficult to dissociate its intended therapeutic effects from off-target effects, which readily leads to adverse reactions. Selexipag is currently the only PGI2 receptor agonist that lacks the PGI2 backbone but demonstrates good selectivity for the PGI2 receptor and definitive efficacy. It has been approved for marketing in multiple countries for the treatment of PAH in adults. Its specificity therapeutic effect is stronger than that of other drugs with similar mechanisms, and it has a longer effect duration. However, its high cost undoubtedly places a significant financial burden on patients with PAH who require long-term treatment. Moreover, although Selexipag has relatively good specificity, it still presents notable adverse reactions, such as headache, facial flushing, nausea, and vomiting. For patients requiring long-term medication, damage resulting from drug accumulation in the body over time, to varying degrees, may diminish their health status and quality of life.

### SUMMARY OF THE INVENTION

### TECHNICAL PROBLEMS

In response to the above issues, the present application provides a diphenyltriazine compound, a preparation method therefor, and a use thereof. The compound exhibits good affinity for the PGI2 receptor and lower adverse reactions, thereby overcoming the drawbacks of current PGI2 analogue and Selexipag.

### TECHNICAL SOLUTIONS

To achieve the above-mentioned object, the present application adopts the following technical solutions:

In a first aspect, the present application provides a diphenyltriazine compound, or a pharmaceutically acceptable salt, or a stable isotope derivative, or an isomer, or a mixture thereof, and the diphenyltriazine compound has a structure represented by Formula I:

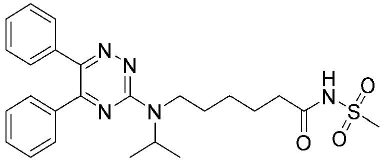

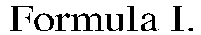

In a second aspect, the present application provides a method for preparing the diphenyltriazine compound, including the following steps:
mixing a compound represented by Formula II, methanesulfonamide, 4-dimethylaminopyridine (DMAP), and carbodiimide in dichloromethane, heating the mixture to 35°C~45°C for reaction for 2~3 hours (h), cooling to 20°C~40 °C, washing the resulting mixture with purified water and followed by hydrochloric acid solution, drying, and removing the solvent to obtain the compound;

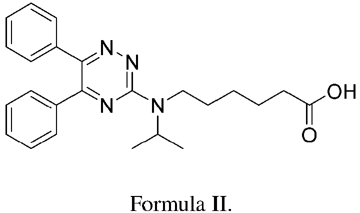

In combination with the second aspect, an equivalent ratio of the methanesulfonamide to the compound represented by Formula II is not less than 1. Preferably, the equivalent ratio of the methanesulfonamide to the compound represented by Formula II is not less than 1.3.

In combination with the second aspect, an equivalent ratio of the 4-dimethylaminopyridine to the compound represented by Formula II is not less than 1. Preferably, the equivalent ratio of the 4-dimethylaminopyridine to the compound represented by Formula II is not less than 1.1.

In combination with the second aspect, an equivalent ratio of the carbodiimide to the compound represented by Formula II is not less than 1. Preferably, the equivalent ratio of the carbodiimide to the compound represented by Formula II is not less than 1.1.

In combination with the second aspect, the amount of the dichloromethane is not limited, provided that it is sufficient to dissolve all the reactants therein.

In combination with the second aspect, the hydrochloric acid solution has a concentration of 1.8 M to 2.2 M (i.e., mol/L).

In combination with the second aspect, anhydrous sodium sulfate is used for drying. Anhydrous sodium sulfate may also be replaced with other desiccants which can dehydrate the organic phase and have no effect on the reactants.

In combination with the second aspect, the method for preparing the compound represented by Formula II includes the following steps:
step 1, mixing glacial acetic acid, benzil, semicarbazide hydrochloride, and purified water, heating the mixture to 100°C~110°C, and maintaining the temperature for reaction for 2 h~2.5 h; cooling to 30°C~40°C, adding purified water, and stirring at 20°C~30°C for reaction for 0.5 h~1 h; performing a first solid-liquid separation to obtain a solid phase and a liquid phase, and placing the solid phase in ethyl acetate to heat to reflux for 2 h~3 h; cooling and performing a second solid-liquid separation to obtain an intermediate II-1;
step 2, mixing phosphorus oxychloride and the intermediate II-1, heating the mixture to 80°C~85°C, stirring until dissolved, and maintaining the temperature for reaction for 1 h~1.5 h; removing the solvent to obtain a first concentrate, adding a mixed solvent of toluene and isopropanol to the first concentrate, stirring to disperse, and performing a third solid-liquid separation to obtain an intermediate II-2;
step 3, adding the intermediate II-2 and 4-(isopropylamino)butanol to a reaction flask sequentially, heating the mixture to 140°C~150°C, and maintaining the temperature for reaction for 12 h~15 h to obtain a first reaction mixture; cooling the first reaction mixture to room temperature, pouring the first reaction mixture into water, and adding ethyl acetate for extraction to obtain a first organic phase; washing the first organic phase with saturated sodium chloride aqueous solution and drying; removing the solvent to obtain a second concentrate, purifying the second concentrate by a first silica gel column chromatography to obtain an intermediate II-3;
step 4, mixing the intermediate II-3 and dichloromethane, cooling the mixture to 0°C~10°C, and adding Dess-Martin periodinane to react at 0°C~10°C for 12 h~15 h to obtain a second reaction mixture; washing the second reaction mixture with saturated sodium bicarbonate solution and followed by saturated sodium chloride aqueous solution to obtain a second organic phase, and drying the second organic phase; removing the solvent to obtain a third concentrate, purifying the third concentrate by a second silica gel column chromatography to obtain an intermediate II-4;
step 5, mixing triethyl phosphonoacetate and tetrahydrofuran, cooling the mixture to 0°C~10°C, and adding sodium hydride to react for 1 h~1.5 h; adding the intermediate II-4 at 0°C~5°C, heating to room temperature and reacting for 2 h~3 h to obtain a third reaction mixture; adding water dropwise to the third reaction mixture to quench the reaction, and concentrating to remove at least 80% of the tetrahydrofuran to obtain a fourth concentrate; adding ethyl acetate to the fourth concentrate for extraction to obtain a third organic phase, washing the third organic phase with saturated sodium chloride aqueous solution and drying; removing the solvent to obtain a fifth concentrate, and purifying the fifth concentrate by a third silica gel column chromatography to obtain an intermediate II-5;
step 6, mixing the intermediate II-5, absolute ethanol, and palladium on carbon, replacing the atmosphere with hydrogen, and reacting at room temperature for 5 h~8 h; removing the palladium on carbon and the solvent to obtain an intermediate II-6; and
step 7, mixing the intermediate II-6, tetrahydrofuran, sodium hydroxide, and purified water, heating the mixture to reflux for 2 h~4 h; concentrating to remove the tetrahydrofuran, adding purified water and ethyl acetate, stirring uniformly and allowing to stand for phase separation to obtain an aqueous phase; adding hydrochloric acid to the aqueous phase to adjust pH to 3~5, and adding methyl tert-butyl ether for extraction to obtain a fourth organic phase; drying the fourth organic phase and removing the solvent to obtain the compound represented by Formula II.

Preferably, in step 1, an equivalent ratio of the semicarbazide hydrochloride to the benzil is greater than 1. Preferably, the equivalent ratio of the semicarbazide hydrochloride to the benzil is greater than 1.4.

Preferably, in step 1, a volume ratio of the glacial acetic acid to the purified water is 2~3:1.

Preferably, in step 1, the amounts of the glacial acetic acid and the purified water are not limited, provided that they are sufficient to dissolve all the reactants therein.

Preferably, in step 2, a mass ratio of the phosphorus oxychloride to the intermediate II-1 is not less than 6.5.

Preferably, in step 2, a volume ratio of the toluene to the isopropanol in the mixed solvent is 1:2.5~3.5.

Preferably, in step 3, an equivalent ratio of the 4-(isopropylamino)butanol to the intermediate II-2 is 3~5:1.

Preferably, in step 3, purifying the second concentrate by a 200-mesh silica gel, with an eluent of dichloromethane-methanol mixed solution in a volume ratio of the dichloromethane to the methanol of 30:1.

Preferably, in step 4, an equivalent ratio of the Dess-Martin periodinane to the intermediate II-3 is 1.5~3:1.

Preferably, in step 4, purifying the third concentrate by a 200-mesh silica gel, with an eluent of n-hexane-ethyl acetate mixed solution in a volume ratio of the n-hexane to the ethyl acetate of 2:1.

Preferably, in step 5, an equivalent ratio of the triethyl phosphonoacetate to the intermediate II-4 is 1~2:1.

Preferably, in step 5, an equivalent ratio of the sodium hydride to the intermediate II-4 is 1~2:1.

Preferably, in step 5, purifying the fifth concentrate by a 200-mesh silica gel, with an eluent of n-hexane-ethyl acetate mixed solution in a volume ratio of the n-hexane to the ethyl acetate of 5:1.

Preferably, in step 6, a mass of the palladium on carbon is 5% to 20% of the mass of the intermediate II-5.

In a third aspect, the present application provides a pharmaceutical composition, which includes a pharmaceutically acceptable carrier, an excipient, and the diphenyltriazine compound, or the pharmaceutically acceptable salt, or the stable isotope derivative, or the isomer, or the mixture thereof as an active ingredient.

In combination with the third aspect, a dosage form of the pharmaceutical composition is pharmaceutically acceptable.

Exemplarily, the dosage form of the pharmaceutical composition includes conventional dosage forms such as tablets, granules, capsules, powders, or injections.

In a fourth aspect, the present application provides a use of the diphenyltriazine compound, or the pharmaceutically acceptable salt, or the stable isotope derivative, or the isomer, or the mixture thereof, or the pharmaceutical composition, in the manufacture of a medicament for treating or preventing pulmonary hypertension, pulmonary arterial hypertension, chronic thromboembolic pulmonary arterial hypertension, Fontan disease and pulmonary hypertension associated with Fontan disease, sarcoidosis and pulmonary hypertension associated with sarcoidosis.

Preferably, the use is in the manufacture of the medicament for treating or preventing pulmonary hypertension, pulmonary arterial hypertension, or chronic thromboembolic pulmonary arterial hypertension.

In a fifth aspect, the present application provides a use of the diphenyltriazine compound, or the pharmaceutically acceptable salt, or the stable isotope derivative, or the isomer, or the mixture thereof, or the pharmaceutical composition, in the manufacture of a medicament for treating or preventing peripheral circulatory disorder, connective tissue disease, chronic kidney disease including glomerulonephritis and diabetic nephropathy at any stage, disease involving organ or tissue fibrosis, respiratory diseases, skin ulcers, digital ulcer, diabetic gangrene, or diabetic foot ulcer.

### ADVANTAGEOUS EFFECTS OF THE INVENTION

The diphenyltriazine compound provided by the present application does not have a PGI2 backbone, has strong selectivity and affinity for a PGI2 receptor, and has good target selectivity as compared with a PGI2 analogue. Experiments have proven that the in-vivo efficacy of the compound is superior to that of Selexipag, and the toxicity of the compound is significantly lower than that of Selexipag. Therefore, the compound can be used as a pulmonary arterial hypertension therapeutic drug having a higher clinical value and development prospect.

### BRIEF DESCRIPTION OF THE DRAWINGS

Fig. 1 is a ¹H-NMR spectrum in Example 1 of the present application.
Fig. 2 is a ¹³C-NMR spectrum in Example 1 of the present application.
Fig. 3 is an HPLC chromatogram in Example 1 of the present application.
Fig. 4 shows EC₅₀ values of Compound A on IP, EP1, EP2, EP3, EP4, DP, FP, and TP targets in Example 2 of the present application.
Fig. 5 shows EC₅₀ values of Selexipag on IP, EP1, EP2, EP3, EP4, DP, FP, and TP targets in Example 2 of the present application.
Fig. 6 shows the effect of Compound A and Selexipag on the proliferation of human pulmonary artery smooth muscle cells in Example 2 of the present application.
Fig. 7 shows the effect of Compound A and Selexipag on the tension of rat pulmonary artery rings in Example 2 of the present application.
Fig. 8 shows the effect of Compound A and Selexipag administered by oral gavage on the survival rate (%) of pulmonary arterial hypertension rats in Example 2 of the present application (Note: Survival rate (%) = (Number of surviving animals in the group / Total number of animals in the group) × 100%).
Fig. 9 shows the effect of Compound A and Selexipag administered by oral gavage on the right ventricular systolic pressure (mmHg) of pulmonary arterial hypertension rats in Example 2 of the present application (*x̅* ± *SD*) (*** indicates P ≤ 0.001 compared with the model control group).
Fig. 10 shows the effect of of Compound A and Selexipag administered by oral gavage on the right ventricular hypertrophy index (%) of pulmonary arterial hypertension rats in Example 2 of the present application (*x̅* ± *SD*) (* indicates P ≤ 0.05 compared with the model control group, *** indicates P ≤ 0.001 compared with the model control group).
Fig. 11 shows the comparison of the percentage of pulmonary arterial media thickness (%) of animals in each group in Example 2 of the present application ( *x̅* ± *SD*) (*** indicates P≤ 0.001 compared with the model control group).
Fig. 12 shows pathological images of rat lungs in each group in Example 2 of the present application, wherein (A) is the normal control group, lung, HE staining, 200×; (B) is the model control group, lung, HE staining, 200×; (C) is the low-dose test substance group, lung, HE staining, 200×; (D) is the medium-dose test substance group, lung, HE staining, 200×; (E) is the high-dose test substance group, lung, HE staining, 200×; and (F) is the positive control group, lung, HE staining, 200×.

### DETAILED DESCRIPTION OF THE INVENTION

In order to make the objectives, technical solutions, and advantages of the present application more apparent, the present application is further described in detail below with reference to specific examples. It should be understood that the specific examples described herein are merely for illustrating the present application and are not intended to limit the scope thereof.

### Terms.

In the preparation methods of the compounds of the present application, "eq" in chemistry means "equivalent". This concept is critical in stoichiometry and chemical reactions, especially when dealing with molar ratios of substances. For example, if 0.3 mol (1 eq) of substance A is used in a certain reaction, and substance B used is 6 times that of substance A (i.e., 6 eq), the amount of substance B used would be 1.8 mol. This representation method facilitates more convenient and accurate calculation of molar ratios of substances in chemical reactions.

In the present application documents, both "eq" and "equivalent" are calculated based on molar ratios, and each step may adopt its independent equivalent standard, for convenience in calculation and experiments.

PGI2 receptor agonists are currently commonly used drugs for the treatment of PAH. However, they have a short biological half-life, poor target selectivity, and are prone to adverse reactions. Selexipag does not have a PGI2 backbone and has relatively better target selectivity, but still exhibits certain adverse reactions. The embodiments of the present application provide a diphenyltriazine compound, and the diphenyltriazine compound has a structure represented by Formula I:

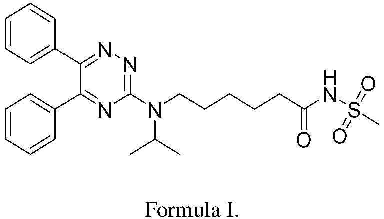

The diphenyltriazine compound does not have a PGI2 backbone, but has strong target selectivity. In-vivo pharmacodynamic experimental results have proven that the efficacy of the compound is superior to that of Selexipag, and toxicological test results have shown that the toxicity of the compound is significantly lower than that of Selexipag. Therefore, the compound has a higher clinical value and development prospect in the treatment of pulmonary arterial hypertension.

The term "isomer" as defined herein means compounds having the same molecular formula but differing in the nature or sequence of bonding of atoms or in the spatial arrangement of atoms. Isomers whose atoms differ in spatial arrangement are referred to as "stereoisomers". Stereoisomers encompass optical isomers, geometric isomers, and conformational isomers.

The compound of the present application may exist in the form of optical isomers. Depending on the configuration of substituents around the chiral carbon atom, these optical isomers are in the "R" or "S" configuration. Optical isomers include enantiomers and diastereomers. Methods for preparing and separating optical isomers are known in the art.

The compounds of the present application may also exist in the form of geometric isomers. The present application contemplates various geometric isomers and mixtures thereof resulting from the distribution of substituents around a carbon-carbon double bond, a carbon-nitrogen double bond, a cycloalkyl group, or a heterocyclic group. Substituents around a carbon-carbon double bond or a carbon-nitrogen bond are designated as the Z or E configuration, while substituents around a cycloalkyl or heterocyclic ring are designated as the cis or trans configuration.

"Isotopes" include all isotopes of atoms occurring in the compounds of the present application. Isotopes include atoms having the same atomic number but different mass numbers. Examples of isotopes suitable for incorporation into the compounds of the present application include isotopes of hydrogen, carbon, nitrogen, oxygen, fluorine, and chlorine, such as, but not limited to, ²H, ³H, ¹³C, ¹⁴C, ¹⁵N, ¹⁸O, ¹⁷O, ³⁵S, ¹⁸F, and ³⁶Cl. Isotopically labeled compounds of the present application can generally be prepared by conventional techniques known to those skilled in the art or by analogous methods to those described in the accompanying examples, using appropriate isotopically labeled reagents in place of the non-isotopically labeled reagents. Such compounds have various potential uses, for example as standards and reagents in determining biological activity. In the case of stable isotopes, such compounds have the potential to beneficially alter biological, pharmacological, or pharmacokinetic properties.

"Pharmaceutically acceptable salt" refers to a salt prepared from a pharmaceutically acceptable base or acid, including inorganic and organic bases or acids. In cases where the compounds of the present application contain one or more acidic or basic groups, the present application also includes their corresponding pharmaceutically acceptable salts. Accordingly, compounds of the present application containing acidic groups may exist as salts and may be used according to the present application, for example as alkali metal salts, alkaline earth metal salts, or ammonium salts, illustratively including sodium, potassium, calcium, magnesium salts, or salts with ammonia or organic amines such as ethylamine, ethanolamine, triethanolamine, or amino acids. Compounds of the present application containing basic groups may exist as salts and may be used according to the present application in the form of their addition salts with inorganic or organic acids. Examples of suitable acids include hydrochloric acid, hydrobromic acid, phosphoric acid, sulfuric acid, nitric acid, methanesulfonic acid, p-toluenesulfonic acid, naphthalenedisulfonic acid, oxalic acid, acetic acid, tartaric acid, lactic acid, salicylic acid, benzoic acid, formic acid, propionic acid, pivalic acid, malonic acid, succinic acid, pimelic acid, fumaric acid, maleic acid, malic acid, sulfamic acid, phenylpropionic acid, gluconic acid, ascorbic acid, isonicotinic acid, citric acid, adipic acid, and other acids known to those skilled in the art. If the compounds of the present application contain both acidic and basic groups in the molecule, the present application also includes internal salts or betaines in addition to the salt forms mentioned. The respective salts can be obtained by conventional methods known to those skilled in the art, for example by contacting them with organic or inorganic acids or bases in a solvent or dispersant, or by anion exchange or cation exchange with other salts.

"Pharmaceutical composition" refers to a composition containing one or more compounds described herein, or pharmaceutically acceptable salts, prodrugs, stable isotope derivatives, isomers or mixtures thereof, as well as other components such as pharmaceutically acceptable carriers and excipients. The purpose of the pharmaceutical composition is to facilitate administration to a living organism and to promote absorption of the active ingredient so as to exert biological activity.

The present application is further illustrated by the following specific examples.

### Example 1.

The present example provides a diphenyltriazine compound, a preparation method and structural characterization thereof.

The preparation method is as follows:

### 1. Preparation of the compound represented by Formula II.

The synthetic route of the intermediate represented by Formula II (chemical name: 6-((5,6-diphenyl-1,2,4-triazin-3-yl)(isopropyl)amino)hexanoic acid) is:

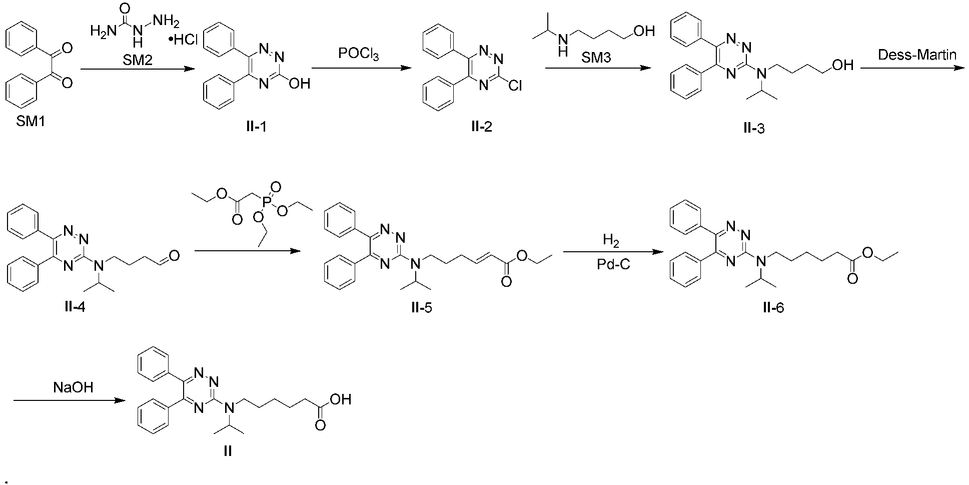

The synthetic method is as follows:
Step 1, glacial acetic acid (2.5 L), benzil (500 g, 1 eq), semicarbazide hydrochloride (889 g, 1.5 eq), and purified water (1 L) were added successively to a reaction flask. The mixture was stirred evenly, heated to 100°C~105°C, and maintained the temperature for reaction for 2.5 h. The mixture was cooled to 30°C~40°C, purified water was added, and the mixture was stirred at 20°C~30°C for 1 h. The mixture was filtered, and the filter cake was suspended in ethyl acetate, heated to reflux, and stirred for 3 h. After cooling to room temperature, the mixture was filtered, and the filter cake was dried under vacuum to give 502 g of Intermediate II-1 (chemical name: 5,6-diphenyl-1,2,4-triazin-3-ol).
Step 2, phosphorus oxychloride (2 L) and Intermediate II-1 (500 g, 1 eq) were added successively to a reaction flask. The mixture was heated to 80°C~85°C, stirred until a clear solution was obtained, and maintained the temperature for reaction for 1.5 h. After concentrating to remove phosphorus oxychloride, a mixed solvent of toluene (500 mL) and isopropanol (1500 mL) was added. The mixture was stirred at room temperature to disperse to obtain a solid, then filtered and dried under vacuum to give 350 g of Intermediate II-2 (chemical name: 3-chloro-5,6-diphenyl-1,2,4-triazine).
Step 3, Intermediate II-2 (100 g, 1 eq) and 4-(isopropylamino)butanol (171.5 g, 3.5 eq) were added successively to a reaction flask. The mixture was heated to 140°C~150°C and maintained the temperature for reaction for 14 h. The reaction mixture was cooled to room temperature, poured into water and extracted with ethyl acetate. The organic phase was washed three times with saturated sodium chloride aqueous solution, and the combined organic phases were dried over anhydrous sodium sulfate and concentrated. The concentrate was purified by silica gel column chromatography (stationary phase: 200-mesh silica gel; eluent (mobile phase): dichloromethane/methanol = 30:1) to give 71 g of Intermediate II-3 (chemical name: 4-((5,6-diphenyl-1,2,4-triazin-3-yl)(isopropyl)amino)butan-1-ol).
Step 4, Intermediate II-3 (70 g, 1 eq) and dichloromethane (700 mL) were added to a reaction flask. The mixture was cooled to 0°C~10°C, and Dess-Martin periodinane (163.8 g, 2 eq) was added portionwise. The mixture was maintained at 0°C~10°C for 14 h. After reaction, the reaction mixture was washed twice with saturated sodium bicarbonate solution and followed by saturated sodium chloride aqueous solution. The organic phase was dried over anhydrous sodium sulfate and concentrated. The concentrate was purified by silica gel column chromatography (stationary phase: 200-mesh silica gel; eluent: n-hexane/ethyl acetate = 2:1) to give 41.3 g of Intermediate II-4 (chemical name: 4-((5,6-diphenyl-1,2,4-triazin-3-yl)(isopropyl)amino)butanal).
Step 5, triethyl phosphonoacetate (29.8 g, 1.2 eq) and tetrahydrofuran (400 mL) were added to a reaction flask. The mixture was cooled to 0°C~10°C, and sodium hydride (4.4 g, 1 eq) was added and stirred for 1 h. Then Intermediate II-4 (40 g, 1 eq) was added at 0°C~5°C. After the addition, the mixture was heated to room temperature to react for 2.5 h. 50 mL of water was added dropwise to the reaction mixture to quench the reaction. The reaction mixture was concentrated to remove most (at least 80%) of the tetrahydrofuran to obtain a concentrate. The concentrate was extracted with 400 mL of ethyl acetate, and the organic phase was washed with saturated sodium chloride aqueous solution, dried over anhydrous sodium sulfate, and concentrated. The concentrate was purified by silica gel column chromatography (stationary phase: 200-mesh silica gel; eluent: n-hexane/ethyl acetate = 5:1) to give 21.3 g of Intermediate II-5 (chemical name: ethyl 6-((5,6-diphenyl-1,2,4-triazin-3-yl)(isopropyl)amino)hex-2-enoate).
Step 6, 21 g of Intermediate II-5, 2.1 g of 10% palladium on carbon, and absolute ethanol (210 mL) were added to a reaction flask. The atmosphere was replaced with hydrogen. The mixture was allowed to react at room temperature for 7 h. The palladium on carbon was removed by filtration, and the filtrate was concentrated to dryness to give 20.2 g of Intermediate II-6 (chemical name: ethyl 6-((5,6-diphenyl-1,2,4-triazin-3-yl)(isopropyl)amino)hexanoate).
Step 7, Intermediate II-6 (20 g, 1 eq), tetrahydrofuran (200 mL), purified water (20 mL), and sodium hydroxide (7.4 g, 4 eq) were added successively to a reaction flask. The mixture was heated to reflux and stirred for 2 h. The tetrahydrofuran was removed by concentration, and purified water (100 mL) and ethyl acetate (100 mL) were added. The mixture was stirred and allowed to stand for phase separation. Hydrochloric acid was added to the aqueous phase to adjust pH to 3~5, then extracted with methyl tert-butyl ether. The organic phase was dried over anhydrous sodium sulfate and concentrated to dryness under reduced pressure to give 16.8 g of the compound represented by Formula II. The ¹H-NMR data are as follows: ¹H-NMR (500 MHz, CDCl₃): δ: 7.510~7.493 (m, 2H), 7.475~7.448 (m, 2H), 7.396~7.364 (m, 1H), 7.317~7.269 (m, 5H), 5.141~5.070 (m, 1H), 3.592 (m, 2H), 2.407~2.362 (m, 2H), 1.779~1.707 (m, 4H), 1.506~1.445 (m, 2H), 1.322~1.308 (m, 6H), ppm.

### 2, Preparation of the compound represented by Formula I.

The compound represented by Formula II (1 g, 1 eq), dichloromethane (20 mL), methanesulfonamide (353 mg, 1.5 eq), DMAP (362 mg, 1.2 eq), and EDCI (569 mg, 1.2 eq) were added sequentially to a reaction flask. The mixture was heated to 35°C~45°C for reaction for 3 h, and cooled to room temperature. The reaction mixture was washed once with purified water, once with 2 M hydrochloric acid solution, and once again with purified water. The organic phase was dried over anhydrous sodium sulfate and concentrated to dryness to give 650 mg of the compound represented by Formula I (chemical name: 6-((5,6-diphenyl-1,2,4-triazin-3-yl)(isopropyl)amino)-N-(methylsulfonyl)hexanamide).

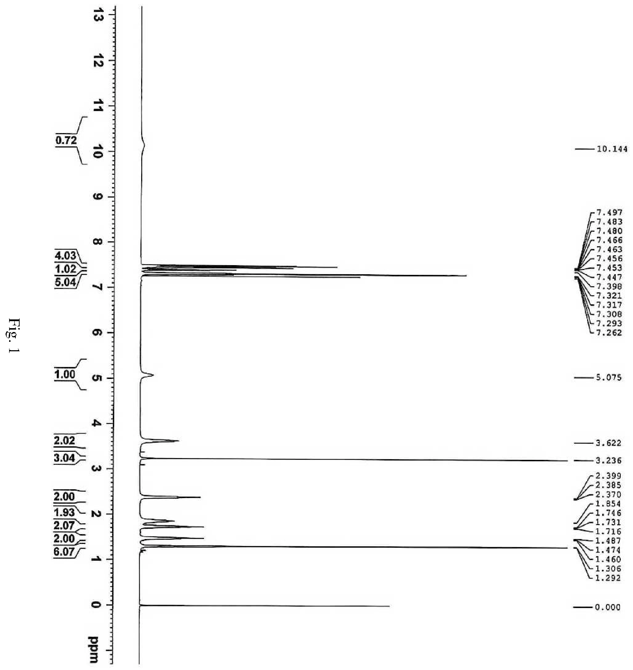

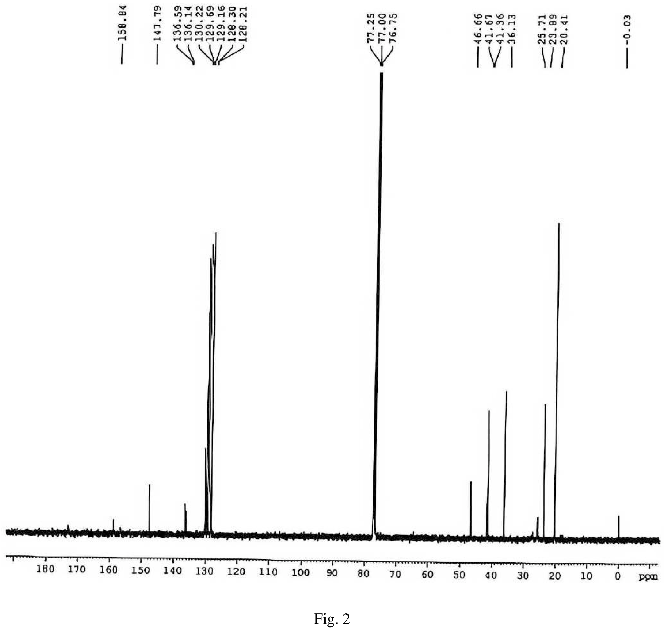

Structural characterization spectra for the compound represented by Formula I are provided in Figs. 1 to 3, with the detailed data given below:

¹H-NMR (500 MHz, CDCl₃) δ: 10.144 (br, 1H), 7.480~7.497 (m, 2H), 7.447~7.466 (m, 2H), 7.398 (m, 1H), 7.262~7.321 (m, 5H), 5.075 (m, 1H), 3.622 (m, 2H), 3.236 (s, 3H), 2.370~2.399 (t, 2H), 1.854~1.716 (m, 4H), 1.460~1.487 (m, 2H), 1.292~1.306 (m, 6H), ppm.

¹³C NMR: (500 MHz, CDCl₃): δ: 20.41, 23.89, 25.71, 36.13, 41.36, 41.67, 46.66, 77.25, 128.21, 128.30, 129.16, 129.69, 130.22, 136.14, 136.59, 147.75, 158.84 ppm.

High-resolution mass spectrum: [M+1]⁺ found (m/z): 482.2210.

High Performance Liquid Chromatography (HPLC) chromatogram: the retention time of the main peak is 5.520 minutes.

Elemental analysis results are shown in Table 1.

**Table 1 Elemental Analysis Results**

| Sample | Test Item | Found/% | | Theoretical/% |
|---|---|---|---|---|
| | | S₁% | S₂% | |
| | Carbon (C%) | 62.44 | 62.36 | 62.35 |
| Compound A | Hydrogen (H%) | 6.56 | 6.53 | 6.49 |
| | Nitrogen (N%) | 14.63 | 14.54 | 14.54 |

From the structural characterization above, Compound A is confirmed to have the structure of Formula I:

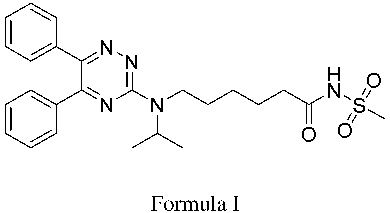

### Example 2.

The example provides the pharmacodynamic study and results for the compound represented by Formula I (hereinafter referred to as "Compound A").

### 1. Evaluation of the Effects of Compound A on IP, EP1, EP2, EP3, EP4, DP, FP, and TP Targets.

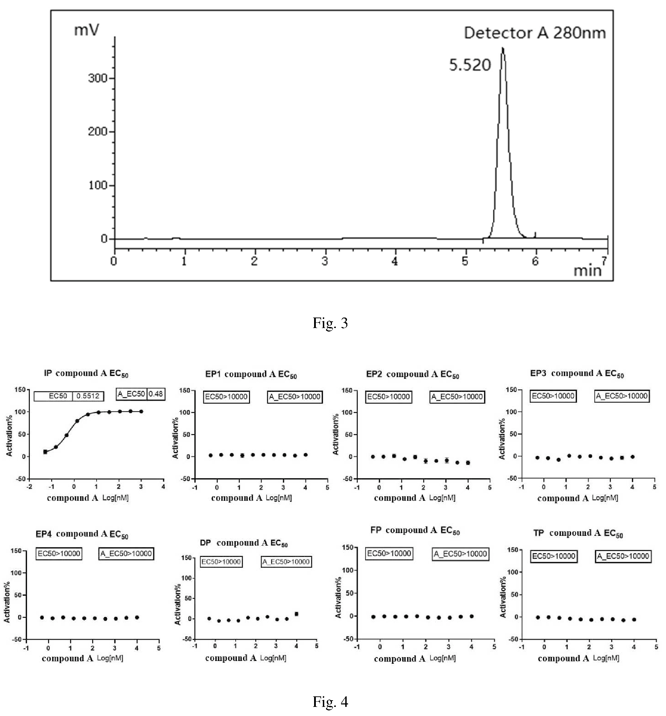

In the experiment, cell lines stably expressing IP, EP1, EP2, EP3, EP4, DP, FP, and TP receptors were employed. The functional activities of Compound A and Selexipag on the eight targets (IP, EP1, EP2, EP3, EP4, DP, FP, and TP) were investigated using HTRF cAMP and HTRF IP1 assays, based on changes in cellular signal intensity. The EC₅₀ values (EC₅₀ generally refers to the dose of an exogenous agent required to cause a 50% change in a biological effect, i.e., the drug concentration that produces 50% of the maximal effect) were calculated from the corresponding concentration-response curves, as shown in Fig. 4.

The results show that Compound A exhibits agonistic activation on the IP target under an initial test concentration of 10000 nM, with an absolute EC₅₀ value of 0.48 nM. For the EP1, EP2, EP3, EP4, DP, FP, and TP targets, Compound A shows no significant agonistic activation under an initial test concentration of 10000 nM, with the absolute EC₅₀ values all greater than 10000 nM.

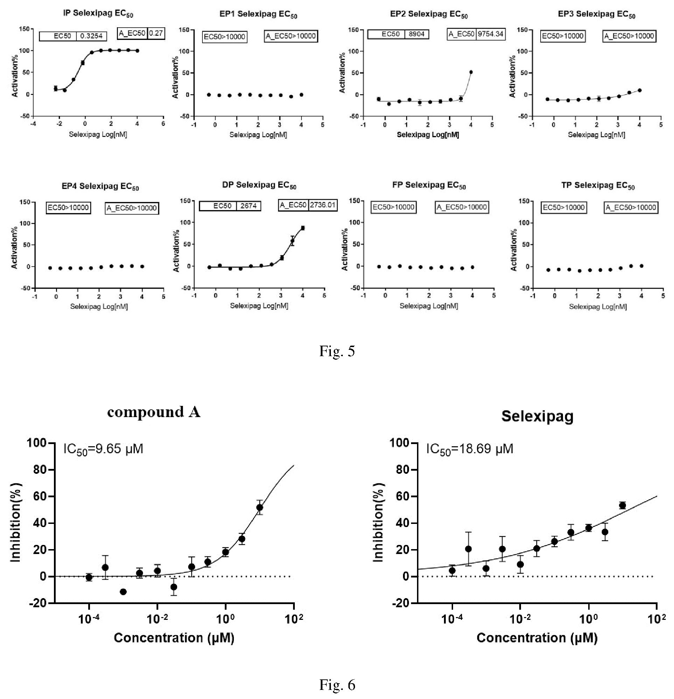

For comparison, the effects of Selexipag on IP, EP1, EP2, EP3, EP4, DP, FP, and TP targets were also tested in the example. The results showed that, under the initial test concentration of 10000 nM, Selexipag exhibited agonistic activation on IP, EP2, and DP targets, with absolute EC50 values of 0.27 nM, 9754.34 nM, and 2736.01 nM, respectively; For the EP1, EP3, EP4, FP, and TP targets, Selexipag exhibited no significant agonistic activation under the same initial concentration of 10000 nM, with the absolute EC₅₀ values all greater than 10000 nM (see Fig. 5).

The above results indicate that Compound A has weaker agonistic activation on the IP receptor than Selexipag, but exhibits stronger target selectivity than Selexipag. Therefore, it can be reasonably inferred that Compound A is less likely to cause adverse reactions similar to those induced by overactivation of the IP receptor by Selexipag (e.g., headache, facial flushing, nausea, vomiting, etc.), and concurrently, the adverse reactions (e.g., muscle pain, etc.) caused by agonism of other prostacyclin receptors are also correspondingly reduced.

### 2. Effect of Compound A on the Proliferation of Human Pulmonary Arterial Smooth Muscle Cells

Human pulmonary arterial smooth muscle cells (HPASMCs) were cultured in smooth muscle cell-specific medium that was incubated in a cell culture incubator at 37 °C with 5 % CO₂ concentration and saturated humidity. The cells were seeded into 96-well plates at a density of 6 × 10³ cells per well with a final volume of 100 µL. After 24 hours, the cells were serum-starved for 24 hours in serum-free medium. Subsequently, Compound A and Selexipag were serially diluted (at concentrations of 10000 nM, 3000 nM, 1000 nM, 300 nM, 100 nM, 30 nM, 10 nM, 3 nM, 1 nM, 0.3 nM, and 0.1 nM) in medium containing 20 ng/mL human Platelet-Derived Growth Factor-BB (hPDGF-BB). The medium in the wells was removed, and 100 µL of the diluted test compounds were added to each well. The CellTiter-Glo (CTG) assay was used to evaluate the inhibitory effects of Compound A and Selexipag on the proliferation of human pulmonary arterial smooth muscle cells, and the half-maximal inhibitory concentrations (IC₅₀) were fitted to evaluate the therapeutic potential of the test compounds for pulmonary arterial hypertension.

The results (see Fig. 6) show that the IC₅₀ values of Compound A and Selexipag for inhibiting PDGF-induced proliferation of pulmonary arterial smooth muscle cells are 9.65 µM and 18.69 µM, respectively. For Compound A, the inhibitory effect is positively correlated with concentrations ranging from 0.3 µM to 10 µM.

In summary, both Compound A and Selexipag exhibit potent inhibitory effects on PDGF-induced proliferation of pulmonary arterial smooth muscle cells. The IC₅₀ value of Compound A is lower than that of Selexipag, indicating that Compound A has stronger inhibition than Selexipag.

### 3. Effect of Compound A on Rat Pulmonary Artery Ring Tension.

SD rats were anesthetized, and the heart and lungs were exposed and isolated, then immersed in oxygenated perfusion solution. After washing the blood away, the isolated heart and lungs were placed in fresh, oxygenated incubation solution. The pulmonary artery was located, and the pulmonary arterial vessels were dissected under a microscope (care was taken not to pull or stretch the pulmonary artery during this process). Residual blood in the pulmonary arterial endothelium was removed to prepare vascular rings with an internal diameter of approximately 2 mm to 3 mm. The vascular rings were mounted onto the hooks of a tension transducer (taking care not to damage the endothelial cells), and the pulmonary arterial branches were immersed in the incubation chamber. The basal tension of the pulmonary arterial branches was adjusted to approximately 0.8 g to1 g via micro-adjustment and allowed to stabilize for 1 h to 2 h. After the basal tension of the pulmonary artery rings was stabilized, a high-K⁺ incubation solution was used to induce an increase in tension of the pulmonary artery rings. Once the tension was stabilized, incubation solutions containing different concentrations of the test compounds were added: Compound A (1 µM, 5 µM, 10 µM, 20 µM, 60 µM) and Selexipag (5 µM, 10 µM, 20 µM, 40 µM, 60 µM), with N=5. The changes in pulmonary artery ring tension were recorded. After the maximum pharmacological effect was observed, the rings were washed with basic incubation solution to allow the pulmonary artery ring tension to return to baseline levels.

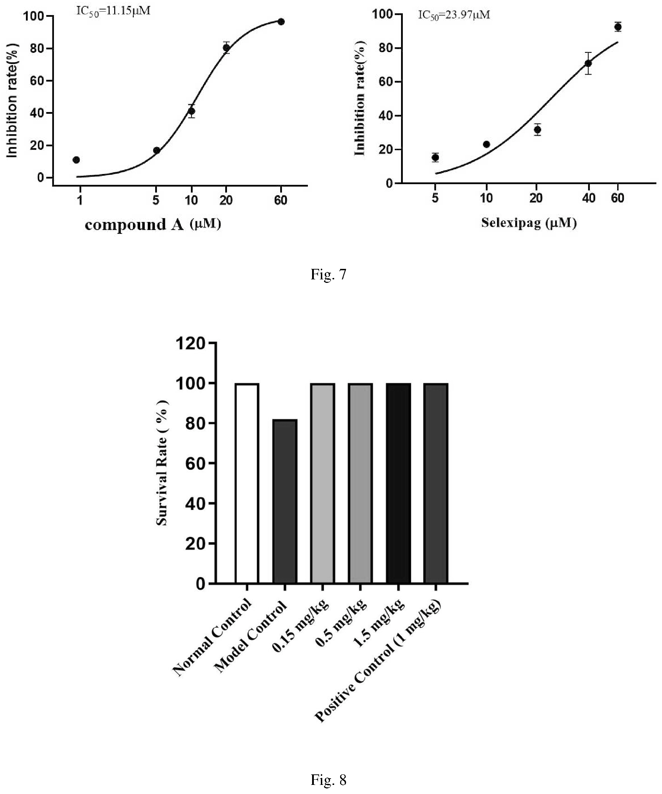

The results (see Fig. 7) show that both Compound A and Selexipag inhibit high-K⁺-induced vasoconstriction of rat pulmonary arteries to varying degrees at all five test concentrations, with half-maximal inhibitory concentrations (IC₅₀) of 11.15 µM and 23.97 µM, respectively. This indicates that the order of inhibition rate from high to low is: Compound A > Selexipag. In conclusion, both Compound A and Selexipag effectively inhibit high-K⁺-induced pulmonary arterial vasoconstriction, and the inhibitory effect of Compound A is greater than that of Selexipag.

### 4. Pharmacodynamic Study of Compound A on Monocrotaline (MCT)-Induced Pulmonary Arterial Hypertension Rat Model.

Sixty-five rats were randomly divided into 6 groups: a normal control group, a model control group, a low-dose test substance group, a medium-dose test substance group, a high-dose test substance group, and a positive control group. The normal control group consisted of 10 rats, while each of the other 5 groups consisted of 11 rats. Except for the normal control group, animals in all other groups were used for model establishment by administration of monocrotaline (60 mg/kg, 5 mL/kg), while animals in the normal control group received intraperitoneal injection of sodium chloride injection (5 mL/kg). Approximately 2 hours after administration of the model-inducing agent, administration of the test substances was initiated in each group.

The normal control group and the model control group were given 0.5% sodium carboxymethyl cellulose (CMC-Na) by oral gavage; the low-dose test substance group was given 0.15 mg/kg of Compound A, the medium-dose test substance group was given 0.5 mg/kg of Compound A, and the high-dose test substance group was given 1.5 mg/kg of Compound A by oral gavage; the positive control group was given 1 mg/kg of Selexipag by oral gavage. All treatments were administered twice daily for 19 consecutive days.

Body weight changes of the rats were recorded, and survival rates were observed. Mean pulmonary artery pressure was measured using right heart catheterization. Histopathological changes in the right ventricular tissue and lung tissue were examined by HE staining. The percentage of medial thickness and the right ventricular hypertrophy index were calculated.

### Results:

### (1) Effect of Compound A on body weight of rats with pulmonary arterial hypertension.

After administration of the model-inducing agent, the body weights of the animals are significantly lower than those in the normal control group. As the experimental period progresses, the mean body weights of animals in each drug administration group increase steadily, whereas the body weights of animals in the model control group decrease more markedly. The data are shown in Table 2.

**Table 2 Effect of Compound A and Selexipag Administered by Oral Gavage on Body Weight (g) of Rats with Pulmonary Arterial Hypertension ( x̅ ± SD ).**

| Day | Normal control group | Model control group | Low-dose test substance group | Medium-dose test substance group | High-dose test substance group | Positive control group |
|---|---|---|---|---|---|---|
| D1 | 299.5±19.8 | 302.6±16.9 | 300.1±11.7 | 297.6±13.5 | 296.9±17.5 | 295.3±19.2 |
| D5 | 338.4±25.7 | 328.5±14.3 | 333.2±7.6 | 327.6±15.4 | 330.4±17.3 | 333.5±18.1 |
| D8 | 363.2±29.0*** | 332.4±17.5 | 354.5±10.0* * | 343.0±15.2 | 347.4±17.5 | 355.0±21.0* * |
| D12 | 386.0±34.3*** | 342.9±23.0 | 369.1±12.3* | 363.4±18.1* | 362.5±24.5 | 376.7±25.0* ** |
| D15 | 389.0±35.7*** | 349.5±29.1 | 381.7±16.0* | 375.2±16.8* | 371.2±27.1* | 383.5±22.8* |
| | | | * | | | * |
| D19 | 416.1±39.9*** | 373.9±28.6 | 406.7±19.0* * | 396.2±20.9 | 391.8±30.9 | 408.2±26.6* * |
| D20 | 421.7±42.1** | 376.3±39.5 | 412.6±18.8* | 401.3±24.0 | 399.3±30.8 | 418.9±28.2* * |

| | | | | | | |
|---|---|---|---|---|---|---|
| Note: Compared with the model control group, "*" indicates *P*≤0.05, "**" indicates *P*≤0.01, and "***" indicates *P*≤0.001. | | | | | | |

### (2) Compound A reduces the mortality rate of rats with pulmonary arterial hypertension.

During the experimental period, the survival rate of animals in the model control group is 82%, whereas no animals died in any of the other groups, giving a survival rate of 100%. The results are shown in Fig. 8.

### (3) Compound A dose-dependently reduces right ventricular systolic pressure, right ventricular hypertrophy index, and pulmonary arterial medial thickness of rats.

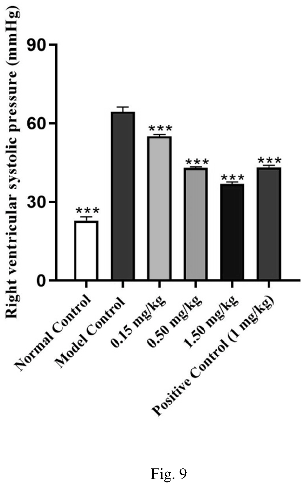

Compared with the right ventricular systolic pressure (RVSP, mmHg) in the model control group, the RVSP values of animals in the low-dose test substance group (0.15 mg/kg), the medium-dose test substance group (0.5 mg/kg), the high-dose test substance group (1.5 mg/kg), and the positive control group (1 mg/kg) are all significantly decreased, with maximum reductions of approximately 15%, 33%, 43%, and 33%, respectively, and all are statistically significant (*P* ≤ 0.001). The data are presented in Fig. 9 and Table 3.

**Table 3 Effect of Compound A and Selexipag Administered by Oral Gavage on Pulmonary Arterial Pressure (mmHg) of Rats with Pulmonary Arterial Hypertension ( x̅ ± SD )**

| Groups | RVSP (mmHg) |
|---|---|
| Normal control group | 22.80±4.29*** |
| Model control group | 64.48±5.35 |
| Low-dose test substance group (0.15 mg/kg) | 55.01±2.54*** |
| Medium-dose test substance group (0.50 mg/kg) | 42.99±1.61*** |
| High-dose test substance group (1.50 mg/kg) | 36.96±2.21*** |
| Positive control group (1 mg/kg) | 43.16±2.66*** |

| | |
|---|---|
| Note: Compared with the model control group, "***" indicates *P*≤0.001. | |

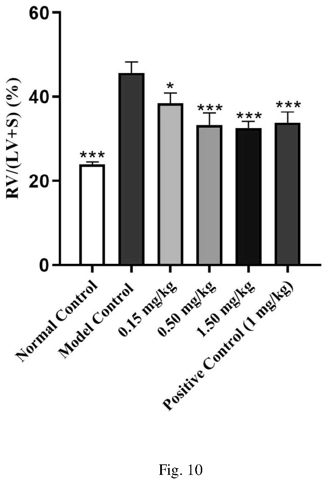

Compared with the animals in the model control group, Compound A significantly reduces the right ventricular hypertrophy index (RV/(LV+S)) of rats at doses of 0.15 mg/kg, 0.5 mg/kg, and 1.5 mg/kg, with maximum reductions of 17%, 27%, and 29%, respectively. Selexipag reduces the right ventricular hypertrophy index of rats by 26% at a dose of 1 mg/kg. As shown in Fig. 10 and Table 4.

**Table 4: Effect of Compound A and Selexipag Administration by Oral Gavage on Right Ventricular Hypertrophy Index (%) of Rats with Pulmonary Arterial Hypertension ( x̅ ± SD).**

| Groups | RV/(LV+S) (%) |
|---|---|
| Normal control group | 23.93±2.03*** |
| Model control group | 45.61±8.75 |
| Low-dose test substance group (0.15 mg/kg) | 38.45±8.01* |
| Medium-dose test substance group (0.50 mg/kg) | 33.29±9.38*** |
| High-dose test substance group (1.50 mg/kg) | 32.57±5.39*** |
| Positive control group (1 mg/kg) | 33.85±8.32*** |

| | |
|---|---|
| Note: Compared with the model control group, "*" indicates *P*≤0.05, "***" indicates *P*≤0.001. | |

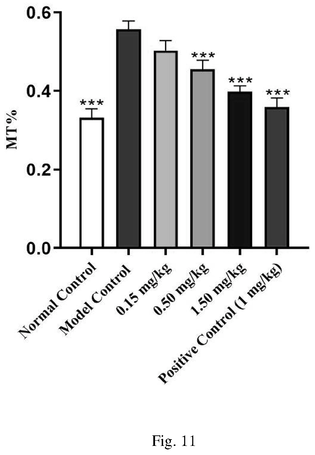

Compared with the model control group, the medial thickness (MT) percentage shows a significant decrease in the Compound A groups at doses of 0.5 mg/kg and 1.5 mg/kg, as well as in the positive control group (*P*≤0.001). The medial thickness percentage in the Compound A group at a dose of 0.15 mg/kg and in the model control group show no significant change. Compared with the Compound A 1.5 mg/kg group, the medial thickness percentage in the positive control group is slightly decreased. As shown in Fig. 11 and Table 5:

**Table 5: Effect of Compound A and Selexipag Administration by Oral Gavage on Medial Thickness Percentage (%) in Pulmonary Artery of Rats with Pulmonary Arterial Hypertension.**

| Groups | Medial Thickness Percentage (%) |
|---|---|
| Normal control group | 0.33±0.07*** |
| Model control group | 0.56±0.07 |
| Low-dose test substance group (0.15 mg/kg) | 0.50±0.08 |
| Medium-dose test substance group (0.50 mg/kg) | 0.46±0.08*** |
| High-dose test substance group (1.50 mg/kg) | 0.40±0.05*** |
| Positive control group (1 mg/kg) | 0.36±0.07*** |

| | |
|---|---|
| Note: Compared with the model control group, "***" indicates *P*≤0.001 | |

### (4) Compound A alleviates pulmonary histopathological injury of rats with pulmonary arterial hypertension.

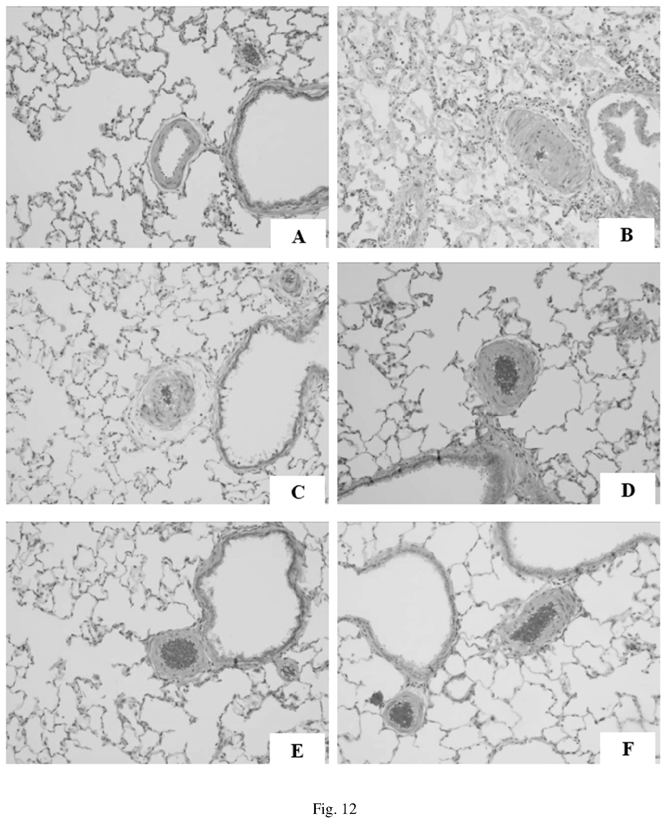

Microscopic examination of animals in the model control group and the drug administration groups reveals various pulmonary and bronchial lesions induced by the pulmonary arterial hypertension model, including alveolar foamy macrophage infiltration, alveolar hemorrhage, alveolar fibrinous exudation, congestion, and pulmonary arterial medial hypertrophy/luminal stenosis. Comprehensively evaluating the lung lesions, the incidence and severity of lung lesions are reduced to certain extents in the groups treated with Compound A and the positive control group as compared with the model control group. Furthermore, the severity of lung lesions in the positive control group is reduced to a certain extent compared with the high-dose test substance group, medium-dose test substance group, and low-dose test substance groups. The results are shown in Fig. 12.

The above results demonstrate that, under the experimental conditions employed, all animals administered by oral gavage twice daily for 19 consecutive days (38 administrations in total), Compound A at doses of 0.15 mg/kg, 0.5 mg/kg, and 1.5 mg/kg, and Selexipag at a dose of 1 mg/kg, significantly reduce pulmonary arterial pressure, the right ventricular hypertrophy index, and pulmonary arterial medial thickness of rats with monocrotaline-induced pulmonary arterial hypertension model, improve survival rates, and have a certain improving effect on lung tissue lesions, exhibiting a dose-response relationship. The efficacy of Compound A at 0.5 mg/kg is comparable to that of the positive control Selexipag (1 mg/kg).

In summary, the diphenyltriazine compound of the present application, or the pharmaceutically acceptable salt, or the stable isotope derivative, or the isomer, or the mixture thereof, exhibit significant treatment or prevention effects in pulmonary hypertension, pulmonary arterial hypertension, chronic thromboembolic pulmonary arterial hypertension, and the like. Furthermore, they exhibit significant effects in Fontan disease and pulmonary hypertension associated with Fontan disease, sarcoidosis and pulmonary hypertension associated with sarcoidosis.

Based on the same mechanism, the diphenyltriazine compound of the present application, or the pharmaceutically acceptable salt, or the stable isotope derivative, or the isomer, or the mixture thereof, are useful in the treatment or prevention of peripheral circulatory disorders, such as chronic arterial occlusion, intermittent claudication, peripheral embolism, vibration syndrome, and Raynaud's disease.

Based on the same mechanism as above, they are useful in the treatment or prevention of connective tissue diseases, such as systemic lupus erythematosus, scleroderma, mixed connective tissue disease, and vasculitis syndrome; they are useful in the treatment or prevention of arteriosclerosis and thrombosis, such as acute cerebral thrombosis and pulmonary embolism; they are useful in the treatment or prevention of thrombocytopenia caused by dialysis, diseases involving fibrosis of organs or tissues, such as kidney diseases including tubulointerstitial nephritis; respiratory diseases such as interstitial pneumonia, (idiopathic) pulmonary fibrosis, and chronic obstructive pulmonary disease; digestive system diseases such as hepatic cirrhosis, viral hepatitis, chronic pancreatitis, and sclerosing gastric cancer; as well as in the prevention or treatment of ulcers, digital ulcers, diabetic gangrene, or diabetic foot ulcers.

### Example 3.

The example provides the toxicological study and results for the compound represented by Formula I (hereinafter referred to as "Compound A").

### 1. Acute Toxicity Study of Compound A Administration by Oral Gavage in Rats.

Twenty-four healthy SD rats (twelve males SD rats and twelve female SD rats) were randomly divided into 4 groups based on body weight. Group 1 served as the vehicle control group (administered at 0 mg/kg), and Groups 2 to 4 were the Compound A low-dose group, Compound A medium-dose group, and Compound A high-dose group, with administered doses of 100 mg/kg, 300 mg/kg, and 1000 mg/kg, respectively. All animals in each group received a single oral gavage administration on Day 1 at a dosing volume of 10 mL/kg, and were observed for 7 days after administration.

One female rat in Group 4 was found moribund on Day 2 and was subsequently euthanized.

Detailed clinical observations revealed that in the Compound A high-dose group (1000 mg/kg), the above-mentioned female rat exhibited loss of righting reflex, cold skin upon touch, ruffled fur, and moribund state on Day 2, which were considered to be toxic reactions induced by Compound A. In the Compound A medium-dose group (300 mg/kg), two female rats showed red nasal discharge on Day 2. The nasal discharge appeared only on Day 2 and subsequently returned to normal. No clear dose-related trend was observed, and although the symptoms were considered related to Compound A, they were not classified as toxic reactions. Compared with body weights during the acclimation period, body weights of female animals in the Compound A high-dose group (1000 mg/kg) decreased on Day 2, which was considered a toxic reaction caused by Compound A. No significant abnormalities in food consumption or gross necropsy findings were observed in each dose group. The maximum tolerated dose (MTD) of Compound A administered by oral gavage in rats was determined to be less than 1000 mg/kg.

The above toxic symptoms were generally consistent with those observed in rats given Selexipag. However, Compound A did not induce severe toxic symptoms such as tail blackening or tail sloughing. The non-lethal dose of Selexipag in rats was 250 mg/kg, and the approximate lethal dose was 500 mg/kg. In conjunction with the pharmacological efficacy data, Compound A has a broader safety dose range in rats than that of Selexipag, and the maximum tolerated dose of Compound A in rats is higher than that of Selexipag.

### 2. 4-Week Repeated Dose Toxicity Study of Compound A in Rats.

One hundred and sixty healthy SD rats (SPF grade) with equal number of males and females, were randomly divided into 8 groups based on sex and body weight. Groups 1 to 4 served as the toxicity study groups (including vehicle control group, Compound A low-dose group, Compound A medium-dose group, and Compound A high-dose group), and each group was consisted of 15 males and 15 females. Groups 5 to 8 served as the toxicokinetic study groups (including vehicle control group, Compound A low-dose group, Compound A medium-dose group, and Compound A high-dose group), and each group was consisted of 5 males and 5 females. Animals in each group were administered Compound A by oral gavage once daily for 4 consecutive weeks at doses of 0 mg/kg (only vehicle), 50 mg/kg, 150 mg/kg, or 500 mg/kg, with a dosing volume of 10 mL/kg.

During the study, animal behavior and physical signs were observed and recorded. Body weight and food consumption were measured once weekly during both the dosing period and the recovery period in Groups 1 to 4, and fundus examination was performed at the end of the dosing period and the recovery period. At the end of the dosing period and the recovery period, clinical pathology examinations (including hematology, coagulation, serum biochemistry, and urinalysis) were conducted on the scheduled necropsy animals. Prior to collection of clinical pathology samples, all scheduled necropsy animals were fasted overnight (≥10 h), and urine samples (approximately 12h) were collected one day in advance for urinalysis. Gross necropsy examination, organ weight measurement, and clinical pathology examination were performed on scheduled necropsy animals at the end of the dosing period and the recovery period.

No animal death was observed during the study. The No Observed Adverse Effect Level (NOAEL) of Compound A was 50 mg/kg. After 28 days of repeated dosing administration at the NOAEL dose, the AUC₍₀₋₂₄₎ values of Compound A were 3784 h×ng/mL (males) and 7016 h×ng/mL (females), respectively. In comparison, the NOAEL of Selexipag was 6 mg/kg, and after 28 days of repeated dosing administration at its NOAEL, the AUC₍₀₋₂₄₎ values of Selexipag were 10 h×ng/mL (males) and 40 h×ng/mL (females), respectively.

Compound A dosing at not less than 500 mg/kg induced salivation and thyroid enlargement in the animals. Compound A dosing at not less than 150 mg/kg caused decreases in body weight, red blood cells (RBC), hemoglobin (HGB), and hematocrit (HCT), and increases in reticulocyte count (RET) and RET%, alanine aminotransferase (ALT), urine volume, liver weight, and histopathological changes including endocrine cell hypertrophy in the pars distalis of the pituitary (males), thyroid follicular cell hypertrophy, and hepatocyte hypertrophy. All changes recovered or showed a trend toward recovery at the end of the recovery period. Among these changes, salivation appeared only on certain examination days in some animals and disappeared after drug withdrawal. In the Compound A medium-dose group (150 mg/kg), the changes in body weight, the decreases in RBC, HGB, and HCT were minimal. The increase in RET/RET% was considered a compensatory response; the increased urine volume is not accompanied by other significant abnormalities in other parameters, which were considered non-adverse reactions related to Compound A. No Compound A-related toxicological reactions were observed in food consumption, ophthalmic examinations, or coagulation parameters in any group.

### Genotoxicity Study of Compound A.

The genotoxicity test was conducted as follows: the test substance (Compound A) and Selexipag were serially diluted by twofold ratios from the top concentration of 1000 µg/well to prepare 8 concentration levels. The plate incorporation method using 6-well plates was employed, with duplicate wells processed in parallel. Negative control (DMSO) and positive control were set up. Parallel experiments were conducted under both conditions with a metabolic activation system (±S9) and without the metabolic activation system (±S9). After incubation for 48h to 72h, the test substance was examined for any precipitate and for the growth of background lawn, and the number of reverse mutation colonies per well was counted. The test results are presented in Tables 6 to 9.

### Criteria for Positive Result

A result may be classified as positive if it satisfies either one or both of the following criteria:
1) At least in one test strain, there is a dose-dependent increase in the number of reverse mutation colonies with or without metabolic activation, and the number of reverse mutation colonies is at least twice that of the negative control group.
2) With or without metabolic activation, one or more dose groups show a significant increase in the number of reverse mutation colonies that is reproducible, and the number of reverse mutation colonies is at least twice that of the negative control group.

When the test substance is tested with two test strains, if either one of the test strains shows a positive result (whether with or without S9 mixture), the test substance can be concluded as a mutagen.

### Criteria for Negative Result.

If the experimental results show no dose-dependent increasing relationship between the number of reverse mutation colonies for all test strains and the dose of test substances, and the peak number of reverse mutation colonies of all strains in all dose groups does not exceed twice that of the negative control group, the test substance can be concluded as a non-mutagen.

The results showed that, under the ±S9 conditions, for TA98 strain and TA100 strain, non-interfering precipitate was observed when the final concentration of Selexipag was not less than 250 µg per well; while for Compound A, non-interfering precipitate was observed when the final concentration was 1000 µg per well. Under the ±S9 conditions, for TA98 strain and TA100 strain, a reduction in background lawn was observed when the final concentration of Selexipag was not less than 250 µg per well; while for Compound A, no abnormal background lawn was observed at any concentration of Compound A. For TA98 strain and TA100 strain, under the ±S9 conditions, the number of reverse mutation colonies in all concentration groups of Compound A and Selexipag did not reach twice that of the negative control group, and no concentration-response relationship was observed. The results were therefore considered negative.

Therefore, Compound A and Selexipag do not exhibit genotoxicity.

**Table 6 Preliminary Results of Bacterial Reverse Mutation Assay with 6-well Plate for Compound A (TA98 Strain).**

| Control/Test substance | | Final concentrat ion (µg/well) | TA98-S9 | | | | TA98+S9 | | | |
|---|---|---|---|---|---|---|---|---|---|---|
| | | | Reverse mutation colony | | Backgro und lawn | Solubil ity | Reverse mutation colony | | Backgro und lawn | Solubil ity |
| | | | Numb er per well | Mea n | | | Numb er per well | Mea n | | |
| Negati ve control | DMSO | 0 | 5 | 4.5 | T0 | P0 | 8 | 7.5 | T0 | P0 |
| | | | 4 | | T0 | P0 | 7 | | T0 | P0 |
| Test substan ce | Compound A | 8 | 6 | 5.0 | T0 | P0 | 11 | 10.0 | T0 | P0 |
| | | | 4 | | T0 | P0 | 9 | | T0 | P0 |
| | | 16 | 5 | 5.0 | T0 | P0 | 8 | 7.5 | T0 | P0 |
| | | | 5 | | T0 | P0 | 7 | | T0 | P0 |
| | | 31 | 9 | 8.5 | T0 | P0 | 8 | 8.5 | T0 | P0 |
| | | | 8 | | T0 | P0 | 9 | | T0 | P0 |
| | | 63 | 4 | 5.5 | T0 | P0 | 7 | 8.0 | T0 | P0 |
| | | | 7 | | T0 | P0 | 9 | | T0 | P0 |
| | | 125 | 8 | 6.5 | T0 | P0 | 12 | 11.5 | T0 | P0 |
| | | | 5 | | T0 | P0 | 11 | | T0 | P0 |
| | | 250 | 7 | 6.5 | T0 | P0 | 9 | 8.0 | T0 | P0 |
| | | | 6 | | T0 | P0 | 7 | | T0 | P0 |
| | | 500 | 5 | 5.5 | T0 | P0 | 10 | 8.5 | T0 | P0 |
| | | | 6 | | T0 | P0 | 7 | | T0 | P0 |
| | | 1000 | 5 | 5.5 | T0 | P1 | 7 | 6.0 | T0 | P1 |
| | | | 6 | | T0 | P1 | 5 | | T0 | P1 |
| Positiv e control | 2-nitrofluorene | 0.2 | 112 | 114. 0* | T0 | P0 | | | | |
| | | | 116 | | T0 | P0 | | | | |
| | 2-aminoanthra cene | 0.6 | | | | | 344 | 348. 0* | T0 | P0 |
| | | | | | | | 352 | | T0 | P0 |

| | | | | | | | | | | |
|---|---|---|---|---|---|---|---|---|---|---|
| Notes: Background lawn: T0 indicates normal. Solubility: P0 indicates normal/no precipitate; P1 indicates non-interfering precipitate under microscopy. The symbol "*" indicates that the number of reverse mutation colonies in the positive control group is 3 times higher than that in the negative control group. | | | | | | | | | | |

**Table 7 Preliminary Results of Bacterial Reverse Mutation Assay with 6-well Plate for Compound A (TA100 Strain).**

| Control/Test substance | | Final concentrat ion (µg/well) | TA100-S9 | | | | TA100+S9 | | | |
|---|---|---|---|---|---|---|---|---|---|---|
| | | | Reverse mutation colony | | Backgro und lawn | Solubil ity | Reverse mutation colony | | Backgro und lawn | Solubil ity |
| | | | Numb er per well | Mea n | | | Numb er per well | Me an | | |
| Negati | DMSO | 0 | 29 | 29.5 | T0 | P0 | 26 | 27.0 | T0 | P0 |
| ve control | | | 30 | | T0 | P0 | 28 | | T0 | P0 |
| Test substan ce | Compound A | 8 | 34 | 23.5 | T0 | P0 | 29 | 29.5 | T0 | P0 |
| | | | 31 | | T0 | P0 | 30 | | T0 | P0 |
| | | 16 | 29 | 29.5 | T0 | P0 | 28 | 27.5 | T0 | P0 |
| | | | 30 | | T0 | P0 | 27 | | T0 | P0 |
| | | 31 | 29 | 28.0 | T0 | P0 | 30 | 29.5 | T0 | P0 |
| | | | 27 | | T0 | P0 | 29 | | T0 | P0 |
| | | 63 | 28 | 27.5 | T0 | P0 | 26 | 27.0 | T0 | P0 |
| | | | 27 | | T0 | P0 | 28 | | T0 | P0 |
| | | 125 | 26 | 27.5 | T0 | P0 | 28 | 27.5 | T0 | P0 |
| | | | 29 | | T0 | P0 | 27 | | T0 | P0 |
| | | 250 | 27 | 28.0 | T0 | P0 | 25 | 25.5 | T0 | P0 |
| | | | 29 | | T0 | P0 | 26 | | T0 | P0 |
| | | 500 | 24 | 24.0 | T0 | P0 | 23 | 22.5 | T0 | P0 |
| | | | 24 | | T0 | P0 | 22 | | T0 | P0 |
| | | 1000 | 21 | 22.5 | T0 | P1 | 24 | 22.0 | T0 | P1 |
| | | | 24 | | T0 | P1 | 20 | | T0 | P1 |
| Positiv e control | Sodium azide | 0.4 | 264 | 266. 0* | T0 | P0 | / | | | |
| | | | 268 | | T0 | P0 | / | | | |
| | 2-aminoanthra | 0.6 | | | | | 444 | 454 | T0 | P0 |
| | cene | | | | | | 464 | * | T0 | P0 |

| | | | | | | | | | | |
|---|---|---|---|---|---|---|---|---|---|---|
| Notes: Background lawn: T0 indicates normal. Solubility: P0 indicates normal/no precipitate; P1 indicates non-interfering precipitate under microscopy. The symbol "*" indicates that the number of reverse mutation colonies in the positive control group is 3 times higher than that in the negative control group. | | | | | | | | | | |

**Table 8 Preliminary Results of Bacterial Reverse Mutation Assay with 6-well Plate for Selexipag (TA98 Strain).**

| Control/Test substance | | Final concentration (µg/well) | TA98-S9 | | | | TA98+S9 | | | |
|---|---|---|---|---|---|---|---|---|---|---|
| | | | Reverse mutation colony | | Backgrou nd lawn | Solu bility | Reverse mutation colony | | Backgrou nd lawn | Solu bility |
| | | | Numbe r per well | Mea n | | | Number per well | M ea n | | |
| Nega tive contr ol | DMSO | 0 | 5 | 4.5 | T0 | P0 | 8 | 7.5 | T0 | P0 |
| | | | 4 | | T0 | P0 | 7 | | T0 | P0 |
| Test subst ance | Selexipag | 8 | 5 | 5.5 | T0 | P0 | 12 | 11 | T0 | P0 |
| | | | 6 | | T0 | P0 | 10 | | T0 | P0 |
| | | 16 | 4 | 4.5 | T0 | P0 | 9 | 10 | T0 | P0 |
| | | | 5 | | T0 | P0 | 11 | | T0 | P0 |
| | | 31 | 4 | 5.5 | T0 | P0 | 10 | 9 | T0 | P0 |
| | | | 7 | | T0 | P0 | 8 | | T0 | P0 |
| | | 63 | 6 | 5.5 | T0 | P0 | 8 | 8.5 | T0 | P0 |
| | | | 5 | | T0 | P0 | 9 | | T0 | P0 |
| | | 125 | 5 | 4.5 | T0 | P0 | 7 | 6.5 | T0 | P0 |
| | | | 4 | | T0 | P0 | 6 | | T0 | P0 |
| | | 250 | 3 | 2.5 | T2 | P1 | 6 | 5.5 | T1 | P1 |
| | | | 2 | | T2 | P1 | 5 | | T1 | P1 |
| | | 500 | NA | - | T3 | P1 | NA | - | T3 | P1 |
| | | | NA | | T3 | P1 | NA | | T3 | P1 |
| | | 1000 | NA | - | T4 | P1 | NA | - | T4 | P1 |
| | | | NA | | T4 | P1 | NA | | T4 | P1 |
| Posit ive contr ol | 2-nitrofluore ne | 0.2 | 112 | 114. 0 * | T0 | P0 | | | | |
| | | | 116 | | T0 | P0 | | | | |
| | 2-aminoan thracene | 0.6 | | | | | 344 | 34 8 * | T0 | P0 |
| | | | | | | | 352 | | T0 | P0 |

| | | | | | | | | | | |
|---|---|---|---|---|---|---|---|---|---|---|
| Notes: NA indicates not counted. Background lawn: T0 indicates normal; T1 indicates slight reduction; T2 indicates moderate reduction; T3 indicates severe reduction; T4 indicates lawn absent. Solubility: P0 indicates normal/no precipitate; P1 indicates non-interfering precipitate under microscopy. The symbol "^{a}" indicates that without S9 and with S9, the positive controls are 2-nitrofluorene (0.2 µg/well) and 2-aminoanthracene (0.6 µg/well), respectively. The symbol "*" indicates that the number of reverse mutation colonies in the positive control group is 3 times higher than that in the negative control group. | | | | | | | | | | |

**Table 9 Preliminary Results of Bacterial Reverse Mutation Assay with 6-well Plate for Selexipag (TA100 Strain).**

| Control/Test substance | | Final concentr ation (µg/well) | TA100-S9 | | | | TA100+S9 | | | |
|---|---|---|---|---|---|---|---|---|---|---|
| | | | Reverse mutation colony | | Backgro und lawn | Solubi lity | Reverse mutation colony | | Backgro und lawn | Solubi lity |
| | | | Num ber per well | Mea n | | | Num ber per well | Me an | | |
| Negati ve contro l | DMSO | 0 | 29 | 29.5 | T0 | P0 | 26 | 27. 0 | T0 | P0 |
| | | | 30 | | T0 | P0 | 28 | | T0 | P0 |
| Test substa nce | Selexipag | 8 | 30 | 29.5 | T0 | P0 | 28 | 29. 0 | T0 | P0 |
| | | | 29 | | T0 | P0 | 30 | | T0 | P0 |
| | | 16 | 29 | 28.0 | T0 | P0 | 29 | 28. | T0 | P0 |
| | | | 27 | | T0 | P0 | 27 | 0 | T0 | P0 |
| | | 31 | 28 | 27.5 | T0 | P0 | 26 | 25. 0 | T0 | P0 |
| | | | 27 | | T0 | P0 | 24 | | T0 | P0 |
| | | 63 | 24 | 24.5 | T0 | P0 | 25 | 24. 0 | T0 | P0 |
| | | | 25 | | T0 | P0 | 23 | | T0 | P0 |
| | | 125 | 23 | 24.0 | T0 | P0 | 20 | 19. 5 | T0 | P0 |
| | | | 25 | | T0 | P0 | 19 | | T0 | P0 |
| | | 250 | 20 | 19.0 | T1 | P1 | 18 | 17. 5 | T1 | P1 |
| | | | 18 | | T1 | P1 | 17 | | T1 | P1 |
| | | 500 | NA | | T3 | P1 | NA | | T3 | P1 |
| | | | NA | | T3 | P1 | NA | | T3 | P1 |
| | | 1000 | NA | | T4 | P1 | NA | | T3 | P1 |
| | | | NA | | T4 | P1 | NA | | T3 | P1 |
| Positi ve contro 1 | Sodium azide | 0.4 | 264 | 266. 0* | T0 | P0 | | | | |
| | | | 268 | | T0 | P0 | | | | |
| | 2-aminoanthr acene | 0.6 | / | | | | 444 | 454 * | T0 | P0 |
| | | | / | | | | 464 | | T0 | P0 |

| | | | | | | | | | | |
|---|---|---|---|---|---|---|---|---|---|---|
| Notes: NA indicates not counted. Background lawn: T0 indicates normal; T1 indicates slight reduction; T3 indicates severe reduction; T4 indicates lawn absent. Solubility: P0 indicates normal/no precipitate; P1 indicates non-interfering precipitate under microscopy. The symbol "^{a}" indicates that without S9 and with S9, the positive controls are sodium azide (0.4 µg/well) and 2-aminoanthracene (0.6 µg/well), respectively. The symbol "*" indicates that the number of reverse mutation colonies in the positive control group is 3 times higher than that in the negative control group. | | | | | | | | | | |

In summary, the NOAEL of Compound A in rats is 100 times its pharmacologically effective dose, whereas the NOAEL of Selexipag is only 6 times its effective dose, indicating that Compound A has a wider safety margin than Selexipag. Furthermore, compared with Selexipag, Compound A does not cause serious adverse reactions after 4 weeks of repeated administration. These results demonstrate that Compound A is safer than Selexipag.

### Example 4.

The example provides a dosage form of tablets for treating pulmonary arterial hypertension, which is prepared by a method comprising: using the compound represented by Formula I as the active ingredient, and preparing tablets with tablet excipients according to conventional processes. The excipients generally include diluents, binders, disintegrants, and the like, to meet the requirements of tablet preparation processes and product quality.

The example also provides other dosage forms of the pharmaceutical composition for treating pulmonary arterial hypertension, including granules, capsules, powders, and injections, all of which are prepared by conventional preparation processes in the art.

The above descriptions are merely preferred examples of the present application and are not intended to limit the present application. Any modifications, equivalent substitutions, or improvements made within the spirit and principles of the present application shall fall within the protection scope of the present application.

## Claims

1. A diphenyltriazine compound, or a pharmaceutically acceptable salt, or a stable isotope derivative, or an isomer, or a mixture thereof, wherein the diphenyltriazine compound has a structure represented by Formula I:

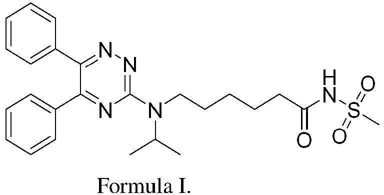

2. A method for preparing the diphenyltriazine compound of claim 1, comprising the following steps:
mixing a compound represented by Formula II, methanesulfonamide, 4-dimethylaminopyridine, and carbodiimide in dichloromethane, heating the mixture to 35°C~45°C for reaction for 2 hours (h)~3 h, cooling to 20°C~40°C, washing the resulting mixture with purified water and followed by hydrochloric acid solution, drying, and removing the solvent to obtain the compound;

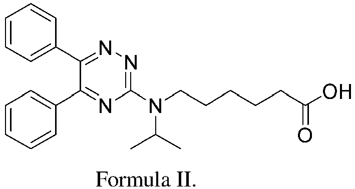

3. The method of claim 2, wherein an equivalent ratio of the methanesulfonamide to the compound represented by Formula II is not less than 1; and/or
an equivalent ratio of the 4-dimethylaminopyridine to the compound represented by Formula II is not less than 1; and/or
an equivalent ratio of the carbodiimide to the compound represented by Formula II is not less than 1.

4. The method of claim 2, wherein the method for preparing the compound represented by Formula II comprises the following steps:
step 1, mixing glacial acetic acid, benzil, semicarbazide hydrochloride, and purified water, heating the mixture to 100°C~110°C, and maintaining the temperature for reaction for 2 h~2.5 h; cooling to 30°C~40°C, adding purified water, and stirring at 20°C~30°C for reaction for 0.5 h~1 h; performing a first solid-liquid separation to obtain a solid phase and a liquid phase, and placing the solid phase in ethyl acetate to heat to reflux for 2 h~3 h; cooling and performing a second solid-liquid separation to obtain an intermediate II-1;
step 2, mixing phosphorus oxychloride and the intermediate II-1, heating the mixture to 80°C~85°C, stirring until dissolved, and maintaining the temperature for reaction for 1 h~1.5 h; removing the solvent to obtain a first concentrate, adding a mixed solvent of toluene and isopropanol to the first concentrate, stirring to disperse, and performing a third solid-liquid separation to obtain an intermediate II-2;
step 3, adding the intermediate II-2 and 4-(isopropylamino)butanol to a reaction flask sequentially, heating the mixture to 140°C~150°C, and maintaining the temperature for reaction for 12 h~15 h to obtain a first reaction mixture; cooling the first reaction mixture to room temperature, pouring the first reaction mixture into water, and adding ethyl acetate for extraction to obtain a first organic phase; washing the first organic phase with saturated sodium chloride aqueous solution and drying; removing the solvent to obtain a second concentrate, purifying the second concentrate by a first silica gel column chromatography to obtain an intermediate II-3;
step 4, mixing the intermediate II-3 and dichloromethane, cooling the mixture to 0°C~10°C, and adding Dess-Martin periodinane to react at 0°C~10°C for 12 h~15 h to obtain a second reaction mixture; washing the second reaction mixture with saturated sodium bicarbonate solution and followed by saturated sodium chloride aqueous solution to obtain a second organic phase, and drying the second organic phase; removing the solvent to obtain a third concentrate, purifying the third concentrate by a second silica gel column chromatography to obtain an intermediate II-4;
step 5, mixing triethyl phosphonoacetate and tetrahydrofuran, cooling the mixture to 0°C~10°C, and adding sodium hydride to react for 1 h~1.5 h; adding the intermediate II-4 at 0°C~5°C, heating to room temperature and reacting for 2 h~3 h to obtain a third reaction mixture; adding water dropwise to the third reaction mixture to quench the reaction, and concentrating to remove at least 80% of the tetrahydrofuran to obtain a fourth concentrate; adding ethyl acetate to the fourth concentrate for extraction to obtain a third organic phase, washing the third organic phase with saturated sodium chloride aqueous solution and drying; removing the solvent to obtain a fifth concentrate, and purifying the fifth concentrate by a third silica gel column chromatography to obtain an intermediate II-5;
step 6, mixing the intermediate II-5, absolute ethanol, and palladium on carbon, replacing the atmosphere with hydrogen, and reacting at room temperature for 5 h~8 h; removing the palladium on carbon and the solvent to obtain an intermediate II-6; and
step 7, mixing the intermediate II-6, tetrahydrofuran, sodium hydroxide, and purified water, heating the mixture to reflux for 2 h~4 h; concentrating to remove the tetrahydrofuran, adding purified water and ethyl acetate, stirring uniformly and allowing to stand for phase separation to obtain an aqueous phase; adding hydrochloric acid to the aqueous phase to adjust pH to 3~5, and adding methyl tert-butyl ether for extraction to obtain a fourth organic phase; drying the fourth organic phase and removing the solvent to obtain the compound represented by Formula II.

5. The method of claim 4, wherein in step 1, an equivalent ratio of the semicarbazide hydrochloride to the benzil is greater than 1; and/or
in step 1, a volume ratio of the glacial acetic acid to the purified water is 2~3:1; and/or
in step 2, a mass ratio of the phosphorus oxychloride to the intermediate II-1 is not less than 6.5; and/or
in step 2, a volume ratio of the toluene to the isopropanol in the mixed solvent is 1:2.5~3.5; and/or
in step 3, an equivalent ratio of the 4-(isopropylamino)butanol to the intermediate II-2 is 3~5; and/or
in step 3, purifying the second concentrate by a 200-mesh silica gel, with an eluent of dichloromethane-methanol mixed solution in a volume ratio of the dichloromethane to the methanol of 30:1; and/or
in step 4, an equivalent ratio of the Dess-Martin periodinane to the intermediate II-3 is 1.5~3:1; and/or
in step 4, purifying the third concentrate by a 200-mesh silica gel, with an eluent of n-hexane-ethyl acetate mixed solution in a volume ratio of the n-hexane to the ethyl acetate of 2:1; and/or
in step 5, an equivalent ratio of the triethyl phosphonoacetate to the intermediate II-4 is 1~2:1; and/or
in step 5, an equivalent ratio of the sodium hydride to the intermediate II-4 is 1~2:1; and/or
in step 5, purifying the fifth concentrate by a 200-mesh silica gel, with an eluent of n-hexane-ethyl acetate mixed solution in a volume ratio of the n-hexane to the ethyl acetate of 5:1; and/or
in step 6, a mass of the palladium on carbon is 5% to 20% of the mass of the intermediate II-5.

6. A pharmaceutical composition, comprising a pharmaceutically acceptable carrier, an excipient, and the diphenyltriazine compound, or the pharmaceutically acceptable salt, or the stable isotope derivative, or the isomer, or the mixture thereof of claim 1 as an active ingredient.

7. The pharmaceutical composition of claim 6, wherein a dosage form of the pharmaceutical composition is pharmaceutically acceptable.

8. The pharmaceutical composition of claim 7, wherein the dosage form of the pharmaceutical composition comprises tablet, granule, capsule, powder or injection.

9. Use of the diphenyltriazine compound, or the pharmaceutically acceptable salt, or the stable isotope derivative, or the isomer, or the mixture thereof of claim 1, or the pharmaceutical composition of any one of claims 6 to 8, in the manufacture of a medicament for treating or preventing pulmonary arterial hypertension, Fontan disease and pulmonary hypertension associated with Fontan disease, sarcoidosis and pulmonary hypertension associated with sarcoidosis.

10. The use of claim 9, wherein the use is in the preparation of a medicament for treating or preventing pulmonary arterial hypertension.

11. The use of claim 10, wherein the pulmonary arterial hypertension is chronic thromboembolic pulmonary arterial hypertension.

12. Use of the diphenyltriazine compound, or the pharmaceutically acceptable salt, or the stable isotope derivative, or the isomer, or the mixture thereof of claim 1, or the pharmaceutical composition of any one of claims 6 to 8, in the manufacture of a medicament for treating or preventing peripheral circulatory disorder, connective tissue disease, chronic kidney disease including glomerulonephritis and diabetic nephropathy at any stage, disease involving organ or tissue fibrosis, digital ulcer, diabetic gangrene, or diabetic foot ulcer.
